(19) Europäisches Patentamt
European Patent Office
Office européen des brevets

(11) EP 1 082 955 A2

(12) EUROPÄISCHE PATENTANMELDUNG

(43) Veröffentlichungstag:
**14.03.2001 Patentblatt 2001/11**

(51) Int. Cl.[7]: **A61K 7/48**

(21) Anmeldenummer: **00118242.7**

(22) Anmeldetag: **04.09.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **08.09.1999 DE 19943429**

(71) Anmelder:
**Beiersdorf Aktiengesellschaft**
**20245 Hamburg (DE)**

(72) Erfinder:
- **Koller, Andreas, Dr.**
  **21035 Hamburg (DE)**
- **Bleckmann, Andreas**
  **22926 Ahrensburg (DE)**
- **Kröpke, Rainer**
  **22869 Schenefeld (DE)**

# (54) Kosmetische und dermatologische Zubereitungen mit einem Gehalt an sulfonierten Kammpolymeren

(57) Kosmetische oder dermatologische Zubereitungen, dadurch gekennzeichnet, daß sie wasserlösliche und/oder wasserdispergierbare Kammpolymere, bestehend aus einer Polymerhauptkette und mit dieser Polymerhauptkette über Estergruppen verknüpften sulfongruppenhaltigen Polyesterseitenarmen, enthalten.



Printed by Xerox (UK) Business Services
2.16.7 (HRS)/3.6

## Beschreibung

Die vorliegende Erfindung betrifft kosmetische und dermatologische Zubereitungen mit einem Gehalt an Kammpolymeren.

Die vorliegende Erfindung betrifft kosmetische und dermatologische Emulsionen, insbesondere hautpflegende kosmetische und dermatologische Emulsionen. In einer vorteilhaften Ausführungsform betrifft die vorliegende Erfindung eine Anwendung, welche es erlaubt, kosmetisch elegante Zubereitungen, insbesondere Emulsionen, mit hervorragenden sensorischen und hautpflegenden Eigenschaften herzustellen.

Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letztlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Epidermis ist ein stratifiziertes Gewebe, in dem die äußere Schicht, die Homschicht (Stratum corneum), den für die Barrierefunktion bedeutenden Teil darstellt. Das heute in der Fachwelt anerkannte Hautmodell von Elias (*P. M. Elias, Structure and Function of the Stratum Corneum Permeability Barrier, Drug Dev. Res.* ***13****, 1988, 97-105*) beschreibt die Hornschicht als Zwei-Komponenten-System, ähnlich einer Ziegelsteinmauer (Ziegelstein-Mörtel-Modell). In diesem Modell entsprechen die Hornzellen (Korneozyten) den Ziegelsteinen, die komplex zusammengesetzte Lipidmembran in den Interzellularräumen entspricht dem Mörtel. Dieses System stellt im wesentlichen eine physikalische Barriere gegen hydrophile Substanzen dar, kann aber aufgrund seiner engen und mehrschichtigen Struktur gleichermaßen auch von lipophilen Substanzen nur schwer passiert werden.

Die vorliegende Erfindung betrifft in einer besonderen Ausführungsform kosmetische oder pharmazeutische Zubereitungen mit vermindertem Klebrigkeitsgefühl, Verfahren zu ihrer Herstellung sowie die Verwendung von Wirkstoffen zur Herabminderung des Klebrigkeitsgefühles kosmetischer Zubereitungen.

Außer ihrer Barrierewirkung gegen externe chemische und physikalische Einflüsse tragen die epidermalen Lipide auch zum Zusammenhalt der Hornschicht bei und haben Einfluß auf die Hautglätte. Im Gegensatz zu den Talgdrüsenlipiden, die keinen geschlossenen Film auf der Haut ausbilden, sind die epidermalen Lipide über die gesamte Hornschicht verteilt.

Das äußerst komplexe Zusammenwirken der feuchtigkeitsbindenden Substanzen und der Lipide der oberen Hautschichten ist für die Regulation der Hautfeuchte sehr wichtig. Daher enthalten Kosmetika in der Regel, neben ausgewogenen Lipidabmischungen und Wasser, wasserbindende Substanzen.

Neben der chemischen Zusammensetzung ist jedoch auch das physikalische Verhalten dieser Substanzen von Bedeutung. Daher ist die Entwicklung von sehr gut bioverträglichen Emulgatoren bzw. Tensiden wünschenswert. Damit formulierte Produkte unterstützen die flüssigkristalline Organisation der Interzellularlipide des Stratum Corneums und verbessern so die Barriereeigenschaften der Hornschicht. Besonders vorteilhaft ist es, wenn deren Molekülbestandteile aus natürlicherweise in der Epidermis vorkommenden Substanzen bestehen.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Übliche kosmetische Darreichungsformen sind Emulsionen. Darunter versteht man im allgemeinen ein heterogenes System aus zwei miteinander nicht oder nur begrenzt mischbaren Flüssigkeiten, die üblicherweise als Phasen bezeichnet werden. Die eine liegt dabei in Form von Tröpfchen vor (disperse oder innere Phase), während die andere Flüssigkeit eine kontinuierliche (kohärente oder innere Phase) bildet. Seltenere Darreichungsformen sind multiple Emulsionen, also solche, welche in den Tröpfchen der dispergierten (oder diskontinuierlichen) Phase ihrerseits Tröpfchen einer weiteren dispergierten Phase enthalten, z.B. W/O/W-Emulsionen und O/W/O-Emulsionen.

Neuere Erkenntnisse führten in letzter Zeit zu einem besseren Verständnis praxisrelevanter kosmetischer Emulsionen. Dabei geht man davon aus, daß die im Überschuß eingesetzten Emulgatorgemische lamellare flüssigkristalline Phasen bzw. kristalline Gelphasen ausbilden. In der Gelnetzwerktheorie werden Stabilität und physikochemische Eigenschaften solcher Emulsionen auf die Ausbildung von viskoelastischen Gelnetzwerken zurückgeführt.

Um die Metastabilität von Emulsionen gewährleisten zu können, sind in der Regel grenzflächenaktive Substanzen, also Emulgatoren, nötig. An sich ist die Verwendung der üblichen kosmetischen Emulgatoren völlig unbedenklich. Dennoch können Emulgatoren, wie letztlich jede chemische Substanz, im Einzelfalle allergische oder auf Überempfindlichkeit des Anwenders beruhende Reaktionen hervorrufen. So ist bekannt, daß bei manchen besonders empfindlichen Personen bestimmte Lichtdermatosen durch gewisse Emulgatoren und gleichzeitige Einwirkung von Sonnenlicht ausgelöst werden.

Es ist möglich, emulgatorfreie Zubereitungen herzustellen, welche beispielsweise in einer wäßrigen Phase dispergierte Öltröpfchen, ähnlich einer O/W-Emulsion, aufweisen. Voraussetzung dafür kann sein, daß die kontinuierliche wäßrige Phase ein die dispergierte Phase stabilisierendes Gelgerüst aufweist und andere Umstände mehr. Solche Systeme werden gelegentlich Hydrodispersionen oder Oleodispersionen genannt, je nachdem, welches die disperse und welches die kontinuierliche Phase darstellt.

Es ist für die kosmetische Galenik aber weder nötig noch möglich, auf Emulgatoren ganz zu verzichten, zumal eine gewisse Auswahl an besonders milden Emulgatoren existiert. Allerdings besteht ein Mangel des Standes der Technik an einer befriedigend großen Vielfalt solcher Emulgatoren, welche dann auch das Anwendungsspektrum entsprechend milder und hautverträglicher kosmetischer Zubereitungen deutlich verbreitern würde.

So war eine Aufgabe der vorliegenden Erfindung, kosmetische bzw. dermatologische Zubereitungen mit hervorragenden hautpflegenden Eigenschaften zur Verfügung zu stellen.

Ein Nachteil insbesondere von O/W-Emulsionen ist oft deren mangelnde Stabilität gegenüber höheren Elektrolytkonzentrationen, was sich in Phasentrennung äußert. Dies kann zwar auch bei W/O-Emulsionen gelegentlich zu Problemen führen, tritt dort aber bei weitem nicht so in den Vordergrund wie bei O/W-Systemen. Zwar läßt sich diesen oft durch geeignete Wahl des Emulgatorsystems in gewissem Maße Abhilfe schaffen, es treten dann aber ebensooft andere Nachteile auf.

Es ist andererseits oft wünschenswert, bestimmte Elektrolyte einzusetzen, um deren sonstige physikalische, chemische bzw. physiologische Eigenschaften nutzen zu können.

Üblicherweise werden die Konzentrationen aller Bestandteile einer kosmetischen oder dermatologischen Zubereitungen in solchen Einheiten wie Gewichts-%, Mol-% und dergleichen angegeben. Aufgrund ihrer mehr oder minder stark ausgeprägten Dissoziation in Kationen und Anionen, oftmals in mehreren Dissoziationsstufen, erscheint es manchmal zweckmäßiger für die Schilderung der vorlegenden Erfindung und ihres technischen Hintergrundes, von der Ionenstärke eines gegebenen Elektrolytes in seiner Lösung auszugehen.

Die Ionenstärke *I* einer Elektrolytlösung ist definiert als

$$I = {}^{1}/_{2} \sum_{i} c_i z_i^2$$

wobei $c_i$ die Konzentrationen der einzelnen Ionensorten (in mol/l) und $z_i$ deren Ladungszahlen darstellen. Die physikalische Einheit der Ionenstärke ist die einer Konzentration (mol/l).

Eine 1-%ige (= 0,17-molare) Kochsalzlösung hat beispielsweise eine Ionenstärke *I* = 0,17.

Eine weitere Aufgabe der vorliegenden Erfindung war es also, Lösungswege zu kosmetischen oder dermatologischen Emulsionen, insbesondere O/W-Emulsionen, aufzudecken, welche gegenüber erhöhten Elektrolytkonzentrationen - bzw. erhöhten Ionenstärken - stabil sind.

Ferner war eine Aufgabe der vorliegenden Erfindung, Zubereitungen zur Verfügung zu stellen, welche den Zustand der Haut deutlich verbessern, insbesondere die Hautrauhigkeit vermindern.

Es ist zwar bekannt, durch Hinzufügen bestimmter Substanzen, beispielsweise einiger ausgewählter Puderrohstoffe, insbesondere Talkum, ein Klebrigkeitsgefühl oder auch Schmierigkeitsgefühl zu reduzieren. Davon abgesehen, daß dieses nur selten vollständig gelingt, wird durch einen solchen Zusatz auch die Viskosität des betreffenden Produktes verändert und die Stabilität verringert.

Aufgabe war daher, all diesen den Nachteilen des Standes der Technik Abhilfe zu schaffen. Insbesondere sollten Produkte mit verringerter Klebrigkeit bzw. Schmierigkeit zur Verfügung gestellt werden. Produkte auf dem Gebiete der pflegenden Kosmetik, der dekorativen Kosmetik und der pharmakologischen Galenik sollten gleichermaßen von den geschilderten Nachteilen des Standes der Technik befreit werden.

Weiterhin war es eine Aufgabe der Erfindung, kosmetische Grundlagen für kosmetische Zubereitungen zu entwickeln, die sich durch gute Hautverträglichkeit auszeichnen.

Ferner war eine Aufgabe der vorliegenden Erfindung, Produkte mit einer möglichst breiten Anwendungsvielfalt zur Verfügung zu stellen. Beispielsweise sollten Grundlagen für Zubereitungsformen wie Reinigungsemulsionen,

Gesichts- und Körperpflegezubereitungen, aber auch ausgesprochen medizinisch-pharmazeutische Darreichungsformen geschaffen werden, zum Beispiel Zubereitungen gegen Akne und andere Hauterscheinungen.

Erstaunlicherweise werden diese Aufgaben gelöst durch kosmetische oder dermatologische Zubereitungen, dadurch gekennzeichnet, daß sie wasserlösliche und/oder wasserdispergierbare Kammpolymere, bestehend aus einer Polymerhauptkette und mit dieser Polymerhauptkette über Estergruppen verknüpften sulfongruppenhaltigen Polyesterseitenarmen, enthalten.

Bevorzugte Ausführungsformen der vorliegenden Erfindung sind kosmetische oder dermatologische Zubereitungen, enthaltend wasserlösliche und/oder wasserdispergierbare Kammpolymere, bestehend aus einer Polymerhauptkette und mit dieser Polymerhauptkette über Estergruppen verknüpften sulfongruppenhaltigen Polyesterseitenarmen, welche wenigstens teilweise durch Natrium und Lithiumgegenionen neutralisiert wurden, wobei das molare Verhältnis von Lithium zu Natrium zwischen 0,1 und 50, bevorzugt zwischen 0,5 und 25, liegt.

Eine weitere bevorzugte Ausführungsform ist die Verwendung wasserlöslicher und/oder wasserdispergierbarer Kammpolymere, bestehend aus einer Polymerhauptkette und mit dieser Polymerhauptkette über Estergruppen verknüpften sulfongruppenhaltigen Polyesterseitenarmen, zur Pflege der Haut.

Eine besonders bevorzugte Ausführungsform der vorliegenden Erfindung ist die Verwendung wasserlöslicher und/oder wasserdispergierbare Kammpolymere, bestehend aus einer Polymerhauptkette und mit dieser Polymerhauptkette über Estergruppen verknüpften sulfongruppenhaltigen Polyesterseitenarmen, welche wenigstens teilweise durch Natrium und Lithiumgegenionen neutralisiert wurden, wobei das molare Verhältnis von Lithium zu Natrium zwischen 0,1 und 50, bevorzugt zwischen 0,5 und 25, liegt, zur Pflege der Haut.

Die erfindungsgemäß eingesetzten Kammpolymere zeichnen sich sowohl durch gute Wasser- und Alkoholverträglichkeit als auch durch günstige Filmeigenschaften und hohem Netzvermögen aus. Zudem sind sie einfach zu formulieren.

Die Grundstruktur der erfindungsgemäßen Kammpolymere folgt im wesentlichen dem folgenden Schema:

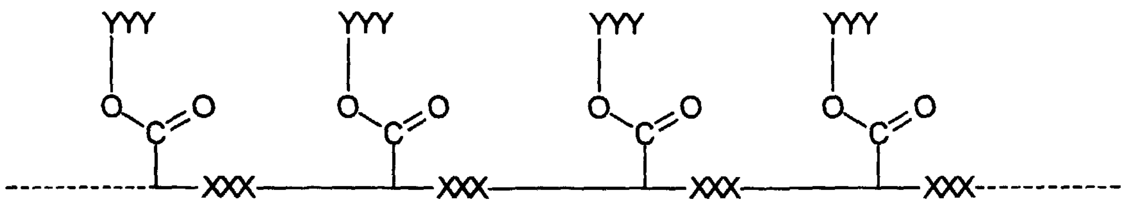

Dabei bedeuten die miteinander verbundenen Gruppierungen mit der Bezeichnung XXX den Grundkörper eines Polymerrückgrates, an welchem über Esterfunktionen Molekülgruppierungen verbunden sind, welche die Bezeichnung YYY tragen. Die Molekülgruppierungen YYY stellen sowohl die vollständigen sulfongruppenhaltigen Polyesterseitenarme der erfindungsgemäßen Kammpolymere dar, können aber auch andere Molekülgruppierungen darstellen.

Dabei besteht die polymere Hauptkette der erfindungsgemäß eingesetzten Kammpolymere bevorzugt aus:

a) polymeren aliphatischen, cycloaliphatischen oder aromatischen Polycarbonsäuren bzw. deren Derivaten wie beispielsweise Polyacrylsäure, Polymethacrylsäure und deren Ester (Ester der beiden Säuren mit aliphatischen, cycloaliphatischen oder aromatischen Alkoholen mit $C_1$ bis $C_{22}$), Maleinsäure, Maleinsäureanhydrid, Fumarsäure und Polynorbonensäure. Die mittleren Molekulargewichte der eingesetzten Polycarbonsäure können zwischen 200 und 2.000.000 g/mol liegen wobei der Bereich von 2.000 - 100.000 g/mol bevorzugt Verwendung findet.

Die mittleren Molekulargewichte der eingesetzten Polycarbonsäure können zwischen 200 und 2.000.000 g/mol liegen wobei der Bereich von 2.000 -100.000 g/mol bevorzugt Verwendung findet.

Die Anbindung der Polyester-Seitenketten erfolgt über eine Estergruppe, die durch die Reaktion einer funktionellen Gruppe der Hauptkette (-COOH im Falle der Polycarbonsäuren oder -OH im Falle der Polyalkohole) mit einer entsprechenden Gruppe des Polyesters (OH im Falle der Polycarbonsäuren und COOH im Falle der Polyalkohole). Selbstverständlich können auch reaktive Derivate der eben angeführten Komponenten zur Reaktion gebracht werden (beispielsweise Anhydride, Ester, Halogenverbindungen und dergleichen mehr).

Die erfindungsgemäß eingesetzten Polyester können sich vorteilhaft durch folgende generische Strukturformeln auszeichnen:

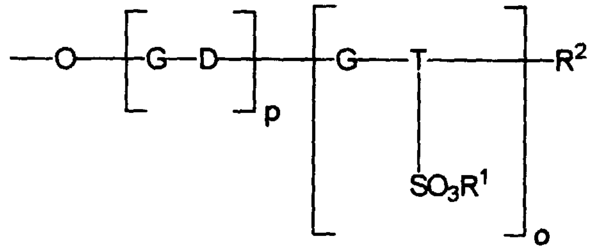

Formel I

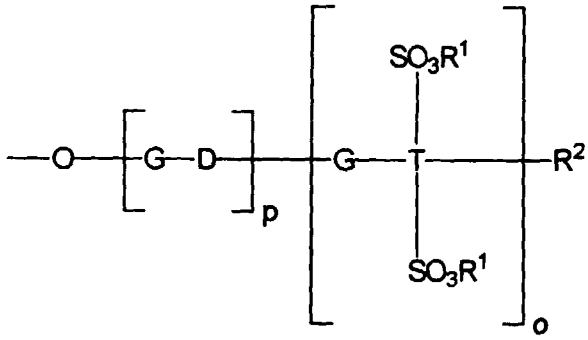

Formel II

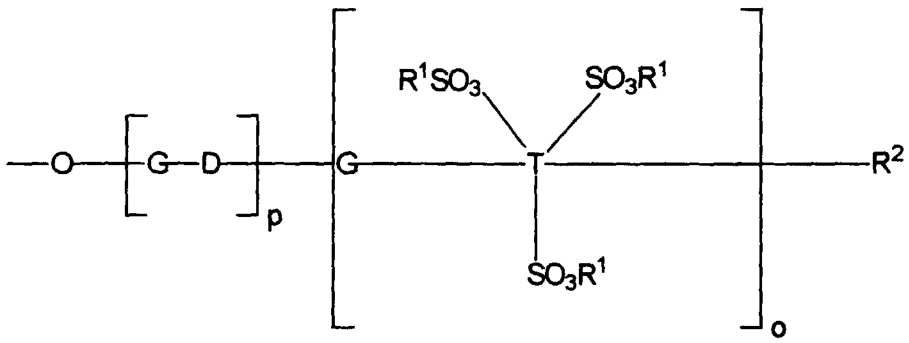

Formel III

usw.

Dabei können p und o so gewählt werden, daß die vorab bezeichneten mittleren Molekulargewichte der eingesetzten Hauptkettenbestandteile zuwege kommen.

Die Polyester-Seitenketten gemäß Formel I - III bestehen vorteilhaft aus:

G : einer mindestens zwei endständige Sauerstoffatome enthaltende aromatische, aliphatische oder cycloaliphatische Organyleinheiten mit einer Kohlenstoffzahl von $C_2$ bis $C_{22}$ oder Abkömmlinge eines Polyglykols der Form $HO-[R^3-O)_k-[R^4O)_m-H$, entsprechend einer Organyleinheit

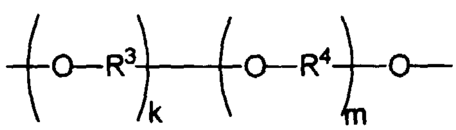

Die Reste $R^3$ und $R^4$ stellen Alkylenreste dar mit einer Kohlenstoffzahl von $C_2$-$C_{22}$, wobei beide Reste nicht notwendigerweise verschieden sein müssen.

Für die Koëffzienten k und m gilt: k+m ≥ 1, wobei k und m ferner so gewählt werden können, daß die vorab

bezeichneten mittleren Molekulargewichte der eingesetzten Hauptkettenbestandteile zuwege kommen.

D : einer mindestens zwei endständige Acylgruppen enthaltenden aromatischen, aliphatischen oder cycloaliphatischen Organyleinheit mit einer Kohlenstoffzahl von $C_2$ bis $C_{22}$, wobei auch Kombinationen aus mehreren verschiedenen Säurekomponenten im beanspruchten Zielmolekül enthalten sein können, beispielsweise eine Organyleinheit des Schemas

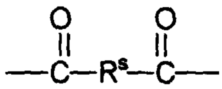

wobei $R^S$ aromatische und lineare oder cyclische, gesättigte oder ungesättigte aliphatische Bifunktionale Reste mit Kohlenstoffzahlen von $C_2$ bis $C_{22}$ darstellen kann. Auch fluorierte oder perfluorierte Acylreste sind dabei im Sinne der Erfindung
oder einer endständige Acylgruppe und eine endständige Oxogruppe enthaltenden aromatischen, aliphatischen oder cycloaliphatischen Organyleinheit mit einer Kohlenstoffzahl von $C_2$ bis $C_{22}$, wobei auch Kombinationen aus mehreren verschiedenen Säurekomponenten im beanspruchten Zielmolekül enthalten sein können, beispielsweise eine Organyleinheit des Schemas

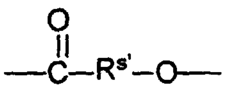

wobei $R^{S'}$ aromatische und lineare oder cyclische, gesättigte oder ungesättigte aliphatische Bifunktionale Reste mit Kohlenstoffzahlen von $C_2$ bis $C_{22}$ darstellen kann. Auch fluorierte oder perfluorierte Acylreste sind dabei im Sinne der Erfindung.

T : eine Verbindung aus der Gruppe der mindestens zwei endständige Acylgruppen enthaltenden sulfonierten aromatischen, aliphatischen oder cycloaliphatischen Organylverbindungen

$R^1$ : Lithium, Natrium, Kalium, Magnesium, Calcium, Ammonium, Monoalkylammonium, Dialkylammonium, Trialkylammonium oder Tetraalkylammonium bedeuten kann, worin die Alkylpositionen der Amine unabhängig voneinander mit $C_1$ bis $C_{22}$-Alkylresten und 0 bis 3 Hydroxylgruppen besetzt sind.

$R^2$ : einen Molekülrest, gewählt aus den Gruppen der

- aromatischen, aliphatischen oder cycloaliphatischen Aminofunktionen: (-NH-$R^5$, -$NR^5_2$ wobei $R^5$ einen Alkyl- oder Arylrest mit $C_1$ bis $C_{22}$ darstellen kann)
- aromatischen, aliphatischen oder cycloaliphatischen Monocarbonsäuregruppen: (-$COOR^6$ wobei $R^6$ ein Alkyl- oder Arylrest darstellt mit $C_1$ bis $C_{200}$)
- über Etherfunktionen verbrückten aromatischen, aliphatischen oder cycloaliphatischen Organylreste: (-O-$R^5$)
- über Etherfunktionen verbrückenden Polyalkoxyverbindungen der Form

$$-O-[R^7-O]_q-[R^8-O]_r-Y$$

Die Reste $R^7$ und $R^8$ stellen vorteilhaft Alkylenreste dar mit einer Kohlenstoffzahl von $C_2$-$C_{22}$, wobei beide Reste nicht notwendigerweise verschieden sein müssen. Der Rest Y kann sowohl Wasserstoff als auch aliphatischer Natur mit $C_1$-$C_{22}$ sein. Für die Koeffizienten q und r gilt: $q+r \geq 1$.
- über Etherfunktionen verbrückenden einfach oder mehrfach ethoxylierten sulfonierten Organylreste oder bevorzugt deren Alkali- oder Erdalkalisalze, wie beispielsweise vorteilhaft gekennzeichnet durch die generische Strukturformel $-(O-CH_2-CH_2)_s-SO_3R^1$ mit $s \geq 1$, und wobei s ferner so gewählt werden kann, daß die vorab bezeichneten mittleren Molekulargewichte der eingesetzen Hauptkettenbestandteile zuwege kommen.

Insbesondere betrifft

$R^1$ : Lithium und/oder Natrium neben gegebenenfalls weiteren Gegenionen, z.B., Kalium, Magnesium, Calcium, Ammonium, Monoalkylammonium, Dialkylammonium, Trialkylammonium oder Tetraalkylammonium bedeuten kann, worin die Alkylpositionen der Amine unabhängig voneinander mit $C_1$ bis $C_{22}$-Alkylresten und 0 bis 3 Hydroxylgruppen besetzt sind.

Die Funktionalität der erfindungsgemäß eingesetzten Komponenten beschränkt sich natürlich nicht auf die Verwendung von OH-Gruppierungen, sondern schließt auch COOH-Endgruppierungen ein oder Mischungen von beiden, wobei auch hier gilt, daß mindestens zwei COOH-Gruppen frei im Molekül vorhanden sein müssen. Reaktive Derivate wie Anhydride, Ester, Epoxide oder Halogenide sind natürlich ebenfalls einsetzbar.

Die mittleren Molekulargewichte der erfindungsgemäßen Kammpolymere können vorteilhaft zwischen 200 und 2.000.000 g/mol liegen, besonders vorteilhaft zwischen 200 und 100.000 g/mol liegen wobei der Bereich von 1.000 - 30.000 g/mol bevorzugt Verwendung findet, ganz besonders vorteilhaft von 5.000 - 15.000 g/mol.

Die erfindungsgemäßen Polyester werden vorteilhaft hergestellt durch Veresterung oder Umesterung der zugrundeliegenden funktionellen Alkoholkomponenten und Diolen mit den Carbonsäuren bzw. deren geeigneten Derivate (beispielsweise Alkylester, Halogenide und dergleichen mehr) in Gegenwart eines Veresterungskatalysators wie Alkalimetallhydroxide, deren -carbonate und Acetate, Erdalkalimetalloxide, -hydroxide, -carbonate und -acetate sowie Alkalimetall- und Erdalkalimetallsalze von Fettsäuren mit 6 bis 22 Kohlenstoffatomen. Weiterhin kommen Titanverbindungen, wie Titanate, metallisches Zinn und organische Zinnverbindungen, wie Mono- und Dialkylzinnderivate als Veresterungskatalysatoren in Betracht. Vorzugsweise wird die Veresterung/Umesterung unter Verwendung von Zinnschliff oder Titantetraisopropylat als Katalysator durchgeführt.

Die Veresterung/Umesterung wird bevorzugt bei Temperaturen von 120 °C bis 280 °C durchgeführt, wobei der entstehende leichter siedende Kondensat (Alkohole oder Wasser) destillativ aus dem Kondensationsprodukt entfernt wird, bevorzugt unter vermindertem Druck bis zu < 0,1 mbar.

Als Edukte für das Polyestergerüst erfindungsgemäßer Kammpolymere können polymere aliphatische, cycloaliphatische oder aromatische Polycarbonsäuren bzw. deren Derivate wie beispielsweise Polyacrylsäure, Polymethacrylsäure und deren Ester (Ester der beiden Säuren mit aliphatischen, cycloaliphatischen oder aromatischen Alkoholen mit $C_1$ bis $C_{22}$), Maleinsäure, Maleinsäureanhydrid, Fumarsäure und Polynorbornensäure eingesetzt werden. Die mittleren Molekulargewichte der einzelnen Polycarbonsäuren können zwischen 200 und 2.000.000 g/mol liegen, wobei der Bereich von 2.000 -100.000 g/mol bevorzugt Verwendung findet.

Auch statistische oder blockartige Copolymere der oben genannten Verbindungsklasse mit anderen vinylischen Monomeren wie beispielsweise Styrol, Acrylamid, $\alpha$-Methylstyrol, Styrol, N-Vinylpyrrolidon, N-Vinylcaprolacton, Acrylamidopropylensulfonsäure und deren Alkali-, Erdalkali- und Ammoniumsalze, MAPTAC (Methacrylamidopropyltrimethylammoniumchlorid), DADMAC, Vinylsulfonsäure, Vinylphosphonsäure, Crotonsäure, Vinylacetamid, Vinylmethylacetamid, Vinylforamid, Acrylsäure oder Methacrylsäurederivate (beispielsweise freie Säure oder Ester), oder Acrylamidderivate oder Vinylacetat können zur Ausbildung der polymeren Hauptkette dienen.

Als Basis für mindestens zwei endständige Sauerstoffatome enthaltende aromatische, aliphatische oder cycloaliphatische Organyleinheiten mit einer Kohlenstoffzahl von $C_2$ bis $C_{22}$ oder Abkömmlinge eines Polyglykols der Form $HO-[R^3-O)_k-[R^4-O)_m-H$, können bifunktionelle Alkoholkomponenten eingesetzt werden.

Dafür eignen sich insbesondere mindestens difunktionelle aromatische, aliphatische oder cycloaliphatische Alkohole mit einer Kohlenstoffzahl von $C_2$ bis $C_{22}$ oder ein Polyglycol der Form $HO-[R^3-O]_k-(R^4-O]_m-H$. Die Reste $R^3$ und $R^4$ stellen Alkylreste dar mit einer Kohlenstoffzahl von $C_2$ bis $C_{22}$, wobei beide Reste gleich oder verschieden sein können. Für die Koeffizienten k und m gilt: $k+m \geq 1$, wobei k und m ferner so gewählt werden können, daß die vorab bezeichneten mittleren Molekulargewichte der eingesetzten Hauptkettenbestandteile zuwege kommen..

Es kann von besonderem Vorteil sein, statt difunktioneller Alkoholkomponenten tri-, tetra- oder allgemein polyfunktionelle Alkoholkomponenten einzusetzen, beispielsweise vorteilhaft gewählt aus der folgenden Gruppe:

Glycerin 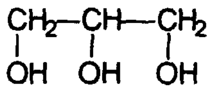 ,

Diglycerin 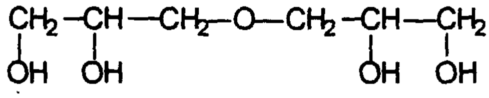 ,

Triglycerin 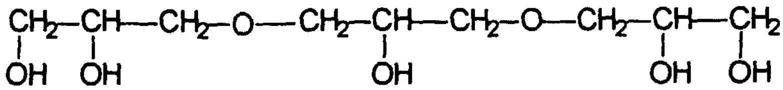 ,

Pentaerythrit 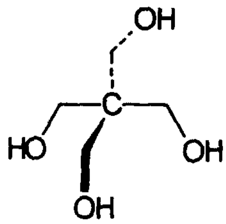 ,

Sorbitol 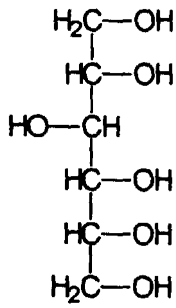 ,

Xylitol 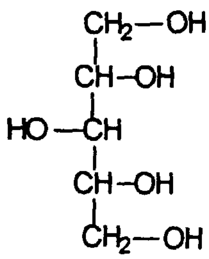

Ascorbinsäure 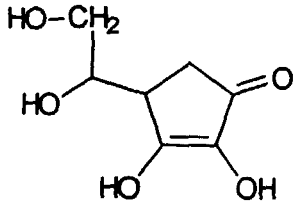

Als Basis für mindestens zwei endständige Acylgruppen enthaltende aromatische, aliphatische oder cycloaliphatische Organyleinheiten mit einer Kohlenstoffzahl von $C_2$ bis $C_{22}$, beispielsweise Organyleinheiten des Schemas

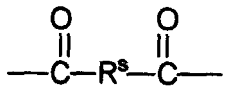
,

können beispielsweise aromatische und lineare oder cyclische, gesättigte oder ungesättigte aliphatische Carbonsäuren mit einer Kohlenstoffzahl von $C_2$ bis $C_{22}$ oder dessen Anhydride eingesetzt werden, beispielsweise Phthalsäure, Isophthalsäure, Naphthalindicarbonsäure, Cyclohexandicarbonsäure, Adipinsäure, Bernsteinsäure, Glutarsäure, Pimelinsäure, Korksäure, Azelainsäure, Sebacinsäure, Brassylsäure eingesetzt werden. Auch Kombinationen aus mehreren verschiedenen Säurekomponenten sind als Monomereinheit im beanspruchten Zielmolekül möglich.

Als sulfongruppenhaltige Monomere eignen sich sulfonierte aromatische, aliphatische oder cycloaliphatische Dialkohole, Disäuren bzw. deren Ester, wie beispielsweise Sulfobernsteinsäure, 5-Sulfoisophthalsäure oder deren Alkali- oder Erdalkalisalze oder Mono-, Di-, Tri- oder Tetraalkylammoniumsalze mit $C_1$ bis $C_{22}$-Alkylresten. Unter den Alkalisalzen sind insbesondere Lithium- und Natriumsalze bevorzugt.

Weiterhin kommen aromatische, aliphatische oder cycloaliphatische Amine mit $C_1$ bis $C_{22}$ Alkyl- bzw. Arylresten und/oder aromatische, aliphatische oder cycloaliphatische Monocarbonsäuren mit $C_1$ bis $C_{200}$ Alkyl- oder Arylresten und/oder Polyalkoxyverbindungen der Form $-O-[R^7-O]_q-(R^8-O]_r-X$, wobei die Reste $R^7$ und $R^8$ Alkylreste, die gleich oder verschieden sein können eine Kohlenstoffzahl von $C_2$ bis $C_{22}$ darstellen und der Rest X sowohl Wasserstoff als auch aliphatischer Natur mit $C_1$ -$C_{22}$ sein kann und die Koeffizienten q und r : $q+r \geq 1$ sind, zum Einsatz.

Ebenso geeignet sind sulfonierte Mono- oder Polyethylenglykole oder bevorzugt deren Alkali- oder Erdalkalisalze: $(H-(O-CH_2-CH_2)_s-SO_3R^1$ mit $s \geq 1$ wobei s ferner so gewählt werden kann, daß die vorab bezeichneten mittleren Molekulargewichte der eingesetzen Hauptkettenbestandteile zuwege kommen.)

Zur Herstellung der erfindungsgemäßen Polyester werden die zur Ausbildung der Seitenkette eingesetzten Alkohole und Säuren bzw. Ester vorteilhaft in den molaren Verhältnissen von 1:1 bis etwa 10:1 (1 bzw. 10 Teile Di- oder Polyol) eingesetzt und der sich bildende Alkohol und Wasser und die Überschußkomponente nach erfolgter Kondensation destillativ entfernt. Im Zielmolekül liegen Alkohol- und Säurekomponenten vorzugsweise im ungefähren stächiometrischen Verhältnis 1:1 vor.

Der Anteil der sulfonsäureresthaltigen Säurekomponenten beträgt 1 bis 99 mol.-%, bevorzugt 10 bis 40 besonders bevorzugt 15 bis 25 mol.-% bezogen auf die Gesamtmenge an Carbonsäuren.

Sehr günstige anwendungstechnische Eigenschaften haben die sulfongruppenhaltigen Polyester der allgemeinen Formel I, wenn als Diolkomponenten 1,2-Propandiol und/oder Diethylenglycol und/oder Cyclohexandimethanol, als Carbonsäuren Isophthalsäure auch mit, 1,3-Cyclohexandicarbonsäure oder auch mit 2,6-Naphthalindicarbonsäure oder auch mit Adipinsäure und als sulfogruppenhaltige Reste 5-Sulfoisophthalsäure-Natrium- und/oder Lithiumsalz, das Natrium- und/oder Lithiumsalz der Isethionsäure eingesetzt werden.

Nachfolgend ist ein Ausschnitt aus einem erfindungsgemäßen Kammpolymermolekül aufgeführt, wobei eine Polyacrylsäurekette das Rückgrat des Kammpolymermoleküls bildet. Die Säurefunktionen sind mit Polyolen verestert, welche ihrerseits mit einer Säurefunktion von Isophthalsäuremolekülen verestert sind. Weitere Polyole, von denen sich Strukturelemente dieses Polymermoleküls herleiten sind Pentaerythritol, 1,2-Propandiol. Als sulfonatgruppenhaltiges Agens, von dem sich Strukturelemente des Polymermoleküls herleiten, dient beispielsweise das 5-Sulfoisophthalsäuredialkylester-Natrium- und/oder Lithiumsalz.

Aus Gründen der Reaktionsführung, welche dem Fachmann bekannt sind, herrscht im Zielpolymer keine absolute Einförmigkeit der Substitution vor, vielmehr ist von einer gewissen statistischen Verteilungsbreite der Substitution auszugehen. Ferner werden bestimmte reaktive Molekülgruppierungen auch zu Vernetzung zweier oder mehrerer Polymerketten zu einem mehr oder weniger komplexen Netzwerk zu beobachten sein, wie es das nachfolgende Molekülschema auch darzustellen versucht.

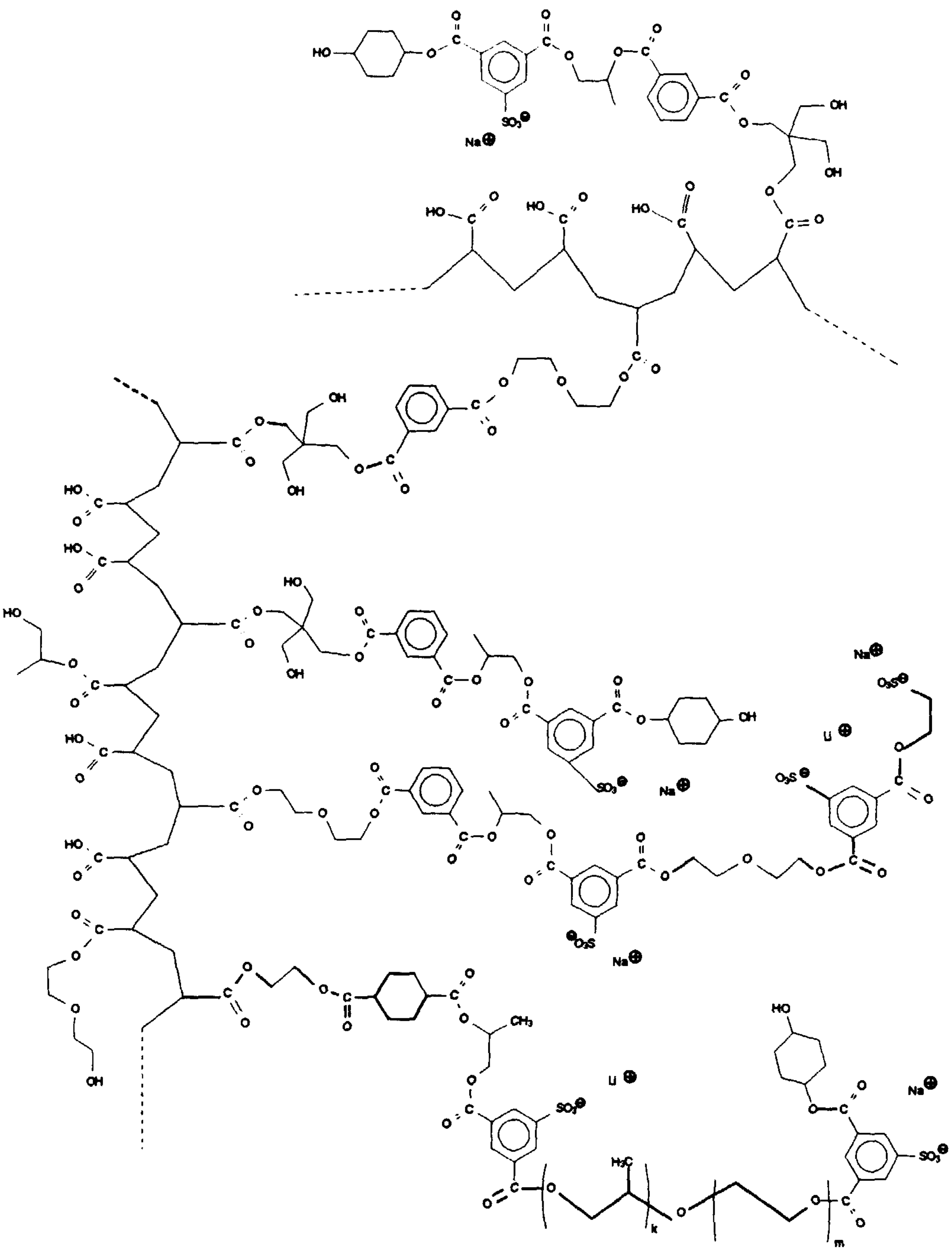

Die erfindungsgemäß einzusetzenden, sulfonhaltigen Polyester sind farblose bis gelbliche, geruchsneutrale Feststoffe. Sie sind in Wasser und Alkoholen gut löslich. Sie können vorteilhaft in kosmetische Zubereitungen zur Festigung der Haare eingearbeitet werden.

Die Herstellung erfindungsgemäßer Kammpolymere erfolgt vorteilhaft, indem ein oder mehrere mehrfunktionelle Alkohole mit einer sulfonsäuregruppenhaltigen, mindestens zwei Carboxylgruppen enthaltenden Substanz, beispielsweise 5-Sulfoisophthalsäuredimethylester-Na-Salz, gegebenenfalls einer weiteren mindestens zwei Carboxylgruppen enthaltenden Substanz und einem Polymer mit einer oder mehreren Polycarbonsäuren, beispielsweise Polyacrylsäure oder Polymethacrylsäure zusammengegeben, erhitzt und den üblichen Aufbereitungsschritten unterworfen werden.

Das oder die Kammpolymere sind in den erfindungsgemäßen Zubereitungen bevorzugt in Mengen von 0,001 - 10,0 Gew.-%, bezogen auf das Gesamtgewicht der Zubereitungen, enthalten. Mengen von 0,01 - 5,0 Gew.-%,

insbesondere von 0,05 - 1,0 Gew.-%, sind besonders bevorzugt.

Die erfindungsgemäßen Zubereitungen zeichnen sich durch erhöhte Stabilität aus, insbesondere wenn sie in Form von Emulsionen, vorteilhaft O/W-Emulsionen, aber auch W/O-Emulsionen, vorliegen. Auch erhöht die Zugabe von erfindungsgemäß verwendeten Kammpolymeren die Stabilität von Emulsionen, insbesondere O/W-Emulsionen aber auch W/O-Emulsionen.

Die erfindungsgemäßen Zubereitungen sind sowohl fließfähig als auch cremeartig formulierbar, besitzen sehr gute kosmetische Eigenschaften, insbesondere was die Klebrigkeit betrifft, und weisen eine sehr gute Hautverträglichkeit sowie Hautpflegeleistung auf.

Erfindungsgemäß ist es möglich und vorteilhaft, den Anteil der Ölphase der erfindungsgemäßen Zubereitungen im Bereich von 0,01 bis 99 Gew.-%, bezogen auf das Gesamtgewicht der Zubereitungen, frei zu wählen.

Als Grundbestandteile der erfindungsgemäßen Zubereitungen können verwendet werden:

- Wasser oder wäßrige Lösungen
- wäßrige ethanolische Lösungen
- natürliche Öle und/oder chemisch modifizierte natürliche Öle und/oder synthetische Öle;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet.

Die Ölphase der Emulsionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkemöl und dergleichen mehr.

Erfindungsgemäß vorteilhaft zu verwendende Fett- und/oder Wachskomponenten können aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse.

Weitere vorteilhafte Fett- und/oder Wachskomponenten sind chemisch modifzierte Wachse und synthetische Wachse, wie beispielsweise die unter den Handelsbezeichnungen Syncrowax HRC (Glyceryltribehenat), Syncrowax HGLC ($C_{16-36}$-Fettsäuretriglycerid) und Syncrowax AW 1C ($C_{18-36}$-Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder $C_{30-50}$-Alkyl Bienenwachs), Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise $C_{20-40}$-Alkylstearat, $C_{20-40}$-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan.

Erfindungsgemäß können die Fett- und/oder Wachskomponenten sowohl einzeln als auch im Gemisch vorliegen.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkybenzoat und 2-Ethylhexylisostearat, Mischungen aus $C_{12-15}$-Alkybenzoat und Isotridecylisononanoat sowie Mischungen aus $C_{12-15}$-Alkybenzoat, 2-Ethylhexylisostearat und Isotridecylisononanoat.

Von den Kohlenwasserstoffen sind Paraffinöl, Cycloparaffin, Squalan, Squalen, hydriertes Polyisobuten bzw. Polydecen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Erfindungsgemäße Emulsionen im Sinne der vorliegenden Erfindung, z.B. in Form einer Hautschutzcrème, einer Hautlotion, einer kosmetischen Milch, beispielsweise in Form einer Sonnenschutzcreme oder einer Sonnenschutzmilch, sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Es ist ebenfalls von Vorteil, von den erfindungsgemäßen Eigenschaften in Form von dekorativen Kosmetika (Make-Up-Formulierungen) Gebrauch zu machen.

Günstig sind auch solche kosmetischen und dermatologischen Zubereitungen, die in der Form eines Sonnenschutzmittels vorliegen. Vorzugsweise enthalten diese neben dem erfindungsgemäß verwendeten Wirkstoff zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescrèmes gewöhnlich UV-A- bzw. UV-B-Filtersubstanzen eingearbeitet.

Vorteilhaft können erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Die UVB-Filter können öllöslich oder wasserlöslich sein. Als öllösliche Substanzen sind z.B. zu nennen:

- 3-Benzylidencampher und dessen Derivate, z.B. 3-(4-Methylbenzyliden)campher,
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;

- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin

Als wasserlösliche Substanzen sind vorteilhaft:

- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, soll selbstverständlich nicht limitierend sein.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Filtersubstanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Kosmetische und/oder dermatologische Zubereitungen im Sinne der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Die erfindungsgemäßen kosmetischen und dermatologischen Zubereitungen können kosmetische Wirk-, Hilfs- und/oder Zusatzstoffe enthalten, wie sie üblicherweise in solchen Zubereitungen verwendet werden, z.B. Antioxidationsmittel, Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Es ist vorteilhaft, den Zubereitungen im Sinne der vorliegenden Erfindung weitere antiirritative oder antientzündliche Wirkstoffe zuzugeben, insbesondere Batylalkohol (α-Octadecylglycerylether), Selachylalkohol (α-9-Octadecenylglycerylether), Chimylalkohol (α-Hexadecylgtycerylether), Bisabolol und/oder Panthenol.

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, ψ-Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Zink und dessen Derivate (z.B. ZnO, $ZnSO_4$) Seien und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Zubereitungen gemäß der vorliegenden Erfindung können auch Verwendung als Grundlage für kosmetische oder dermatologische Desodorantien bzw. Antitranspirantien finden. Alle für Desodorantien bzw. Antitranspirantien gängigen Wirkstoffe können vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure.

Keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol) sowie die in den Patentoffenlegungsschriften DE-37 40 186, DE-39 38 140, DE-42 04 321, DE-42 29 707, DE-43 09 372, DE-44 11 664, DE-195 41 967, DE-195 43 695, DE-195 43 696, DE-195 47 160, DE-196 02 108, DE-196 02 110, DE-196 02 111, DE-196 31 003, DE-196 31 004 und DE-196 34 019 und den Patentschriften DE-42 29 737, DE-42 37 081, DE-43 24 219, DE-44 29 467, DE-44 23 410 und DE-195 16 705 beschriebenen Wirkstoffe bzw. Wirkstoffkombinationen. Auch Natriumhydrogencarbonat ist vorteilhaft zu verwenden.

Die Menge solcher Wirkstoffe (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen im Sinne der vorliegenden Erfindung zur Behandlung und Pflege der Haare, die den erfindungsgemäß verwendeten Wirkstoff enthalten, können als Emulsionen vorliegen, die vom nicht-ionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen.

Die Wasserphase der kosmetischen Zubereitungen im Sinne der vorliegenden Erfindung kann auch Gelcharakter aufweisen, die neben einem wirksamen Gehalt am erfindungsgemäß eingesetzten Substanzen und dafür üblicherweise verwendeten Lösungsmitteln, bevorzugt Wasser, noch weitere organische Verdickungsmittel, z.B. Gummiarabikum, Xanthangummi, Natriumalginat, Stärke und Stärkederivate (z.B. Distärkephosphat), Cellulose, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise organisch modifizierte oder auch unmodifizierte Hectorite, Bentonite, oder dergleichen, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Ferner kann es von Vorteil sein, Zubereitungen gemäß der Erfindung grenz- bzw. oberflächenaktive Agentien zuzufügen, beispielsweise kationische Emulgatoren wie insbesondere quaternäre Tenside.

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid,

Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Vorteilhaft ist auch, kationische Polymere (z.B. Jaguar C 162 [Hydroxypropyl Guar Hydroxypropyltrimonium Chloride] bzw. modifizierten Magnesiumaluminiumsilikaten (z.B. Quaternium-18-Hectorit, welches z. B. unter der Handelsbezeichnung Bentone® 38 bei der Firma Rheox erhältlich ist, oder Stearalkonium Hectorit, welches z. B. unter der Handelsbezeichnung Softisan® Gel bei der Hüls AG erhältlich ist), einzusetzen.

Erfindungsgemäße Zubereitungen können vorteilhaft auch Ölverdickungsmittel enthalten, um die taktilen Eigenschaften der Emulsion und die Stiftkonsistenz zu verbessern. Vorteilhafte Ölverdickungsmittel im Sinne der vorliegenden Erfindung sind beispielsweise weitere Feststoffe, wie z. B. hydrophobe Siliciumoxide des Typs Aerosil®, welche von der Degussa AG erhältlich sind. Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 972, Aerosil® R 974 und/oder Aerosil® R976.

Ferner sind auch sogenannte Metallseifen (d. h. die Salze höherer Fettsäuren mit Ausnahme der Alkalisalze) vorteilhafte Ölverdickungsmittel im Sinne der vorliegenden Erfindung, wie beispielsweise Aluminium-Stearat, Zink-Stearat und/oder Magnesium-Stearat.

Ebenfalls vorteilhaft ist, Zubereitungen gemäß der Erfindung amphotere bzw. zwitterionischen Tensiden (z.B. Cocoamidopropylbetain) und Moisturizern (z.B. Betain) zuzusetzen. Vorteilhaft zu verwendende amphotere Tenside sind beispielsweise Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat, N-Alkylaminosäuren, beispielsweise Aminopropylalkylglutamid, Alkylaminopropionsäure, Natriumalkylimidodipropionat und Lauroamphocarboxyglycinat.

Die Menge der ober- bzw. grenzflächenaktiven Substanzen (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Erfindungsgemäße Zubereitungen können auch Wirkstoffe (eine oder mehrere Verbindungen) enthalten, welche gewählt werden aus der Gruppe: Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin $B_1$, das Vitamin $B_{12}$ das Vitamin $D_1$, aber auch Bisabolol, ungesättigte Fettsäuren, namentlich die essentiellen Fettsäuren (oft auch Vitamin F genannt), insbesondere die $\gamma$-Linolensäure, Ölsäure, Eicosapentaänsäure, Docosahexaänsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschäl oder Johannisbeerkernöl, Fischöle, Lebertran aber auch Ceramide und ceramidähnliche Verbindungen und so weiter. Vorteilhaft ist es auch, die Wirkstoffe aus der Gruppe der rückfettenden Substanzen zu wählen, beispielsweise Purcellinöl, Eucerit® und Neocerit®.

Die Menge solcher Wirkstoffe (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die nachfolgenden Beispiele sollen die vorliegende Erfindung verdeutlichen.

## (A) Herstellungsbeispiele

Herstellungsbeispiel 1

| Edukt | Masse (g) |
|---|---|
| Isophthalsäure | 265,81 |
| 5-Sulfoisophthalsäuremethylester, Na-Salz | 100,52 |
| 5-Sulfoisophthalsäure, Li-Salz | 22,92 |
| Isethionsäure, Na-Salz | 10,96 |
| Polyacrylsäure ($M_n$ =2.000 g/mol) | 3,00 |
| Natriumcarbonat | 0,60 |

(fortgesetzt)

| Edukt | Masse (g) |
|---|---|
| Titantetraisopropylat | 0,60 |
| 1,2-Propandiol | 195,40 |
| Diethylenglycol | 166,95 |

Herstellweise:

In einem 2 l Vierhalskolben mit KPG-Rührer, Innenthermometer, Gaseinleitungsrohr und Destillationsbrücke werden 1,2-Propandiol, Diethylenglykol, 5-Sulfoisophthalsäuremethylester-Na-Salz, 5-Sulfoisophthalsäure-Li-Salz, Natriumcarbonat und Titantetraisopropylat vorgelegt, und bei 180 °C 5 Stunden lang umgeestert. Das dabei entstehende Methanol und Reaktionswasser werden bei Normaldruck abdestilliert. Anschließend werden das Natriumsalz der Isethionsäure, Isophthalsäure und Polyacrylsäure zur Reaktionsmischung hinzugegeben, hernach gut mit $N_2$ inertisiert. Die Reaktionsmischung wird dann langsam auf 220 °C aufgeheizt und solange bei dieser Temperatur gehalten, bis kein weiteres Kondensat überdestilliert. In einem Zeitraume von drei Stunden wird dann, von Normaldruck ausgehend, der Innendruck des Reaktionsgefäßes auf Ölpumpenvakuum abgesenkt. Nach weiteren 60 Minuten wird mit $N_2$ belüftet und die heiße Polymerschmelze ausgetragen.

<u>Herstellungsbeispiel 2</u>

| Edukt | Mol (mmol) |
|---|---|
| 1,3-Cyclohexandicarbonsäure | 132,80 |
| Terephthalsäuredimethylester | 95,12 |
| 5-Sulfoisophthalsäuremethylester, Na-Salz | 75,20 |
| 5-Sulfoisophthalsäure, Li-Salz | 75,20 |
| Polyacrylsäure ($M_n$ =5.000 g/mol) | 7,00 |
| Natriumcarbonat | 0,60 |
| Titantetraisopropylat | 0,80 |
| 1,2-Propandiol | 195,40 |
| Diethylenglycol | 166,95 |

Herstellweise:

In einem 2 l Vierhalskolben mit KPG-Rührer, Innenthermometer, Gaseinleitungsrohr und Destillationsbrücke werden 1,2-Propandiol, Diethylenglykol, 5-Sulfoisophthalsäuremethylester-Na-Salz, 5-Sulfoisophthalsäure-Li-Salz, Natriumcarbonat und Titantetraisopropylat vorgelegt, und bei 180 °C 5 Stunden lang umgeestert. Das dabei entstehende Methanol und Reaktionswasser werden bei Normaldruck abdestilliert. Anschließend werden das Natriumsalz der Isethionsäure, 1,3-Cyclohexandicarbonsäure und Polyacrylsäure zur Reaktionsmischung hinzugegeben, hernach gut mit $N_2$ inertisiert. Die Reaktionsmischung wird dann langsam auf 220 °C aufgeheizt und solange bei dieser Temperatur gehalten, bis kein weiteres Kondensat überdestilliert. In einem Zeitraume von drei Stunden wird dann, von Normaldruck ausgehend, der Innendruck des Reaktionsgefäßes auf Ölpumpenvakuum abgesenkt. Nach weiteren 60 Minuten wird mit $N_2$ belüftet und die heiße Polymerschmelze ausgetragen.

Herstellungsbeispiel 3

| Edukt | Masse (g) |
|---|---|
| 2,6-Naphthalindicarbonsäure | 172,95 |
| 5-Sulfoisophthalsäuremethylester, Na-Salz | 55,52 |
| 5-Sulfoisophthalsäure, Li-Salz | 97,13 |
| Isophthalsäure | 55,00 |
| Poly[acrylsäure-co-methacrylsäuremethylester $M_n$ = 5.000 g/mol) * | 3,00 |
| Natriumcarbonat | 0,60 |
| Titantetraisopropylat | 0,60 |
| 1,2-Propandiol | 188,40 |
| Diethylenglycol | 155,12 |

Herstellweise:

In einem 2 l Vierhalskolben mit KPG-Rührer, Innenthermometer, Gaseinleitungsrohr und Destillationsbrücke werden 1,2-Propandiol, Diethylenglykol, 5-Sulfoisophthalsäuremethylester-Na-Salz, 5-Sulfoisophthalsäure-Li-Salz, Natriumcarbonat und Titantetraisopropylat vorgelegt, und bei 180 °C 5 Stunden lang umgeestert. Das dabei entstehende Methanol und Reaktionswasser werden bei Normaldruck abdestilliert. Anschließend werden 2,6-Naphthalindicarbonsäure, Isophthalsäure und Poly[acrylsäure-co-methacrylsäuremethylester zur Reaktionsmischung hinzugegeben, hernach gut mit $N_2$ inertisiert. Die Reaktionsmischung wird dann langsam auf 220 °C aufgeheizt und solange bei dieser Temperatur gehalten, bis kein weiteres Kondensat überdestilliert. In einem Zeitraume von drei Stunden wird dann, von Normaldruck ausgehend, der Innendruck des Reaktionsgefäßes auf Ölpumpenvakuum abgesenkt. Nach weiteren 75 Minuten wird mit $N_2$ belüftet und die heiße Polymerschmelze ausgetragen.

Herstellungsbeispiel 4

| Edukt | Mol (mmol) |
|---|---|
| Adipinsäure | 172,95 |
| 5-Sulfoisophthalsäuremethylester, Na-Salz | 55,52 |
| 5-Sulfoisophthalsäure, Li-Salz | 97,13 |
| Isophthalsäure | 55,00 |
| Polyacrylsäure ($N_n$ = 5.000 g/mol) | 3,00 |
| Natriumcarbonat | 0,60 |
| Titantetraisopropylat | 0,60 |
| Diethylenglycol | 400,00 |

Herstellweise:

In einem 2 l Vierhalskolben mit KPG-Rührer, Innenthermometer, Gaseinleitungsrohr und Destillationsbrücke werden Diethylenglykol, 5-Sulfoisophthalsäuremethylester-Na-Salz, 5-Sulfoisophthalsäure-Li-Salz, Natriumcarbonat, Titantetraisopropylat, Isophthalsäure, Polyacrylsäure und Adipinsäure vorgelegt, und langsam auf 200 °C aufgeheizt. Das dabei entstehende Methanol und Reaktionswasser werden bei Normaldruck abdestilliert. Anschließend wird ausreichend mit $N_2$ inertisiert und die Reaktionsmischung langsam auf 250 °C aufgeheizt und solange bei dieser Temperatur gehalten, bis kein Kondensat mehr überdestillliert. In einem Zeitraume von drei Stunden wird dann, von Normaldruck ausgehend, der Innendruck des Reaktionsgefäßes auf Ölpumpenvakuum abgesenkt. Nach weiteren 75 Minuten wird mit $N_2$ belüftet und die heiße Polymerschmelze ausgetragen.

Herstellungsbeispiel 5

| Edukt | Mol (mmol) |
|---|---|
| 1,4-Cyclohexandicarbonsäure | 172,95 |
| 5-Sulfoisophthalsäuremethylester, Na-Salz | 55,52 |
| 5-Sulfoisophthalsäure, Li-Salz | 97,13 |
| Isophthalsäure | 55,00 |
| Polyacrylsäure ($M_n$ = 5.000 g/mol) | 3,00 |
| Cyclohexandimethanol | 150,12 |
| Natriumcarbonat | 0,60 |
| Titantetraisopropylat | 0,60 |
| Diethylenglycol | 400,00 |

Herstellweise:

In einem 2 l Vierhalskolben mit KPG-Rührer, Innenthermometer, Gaseinleitungsrohr und Destillationsbrücke werden Cyclohexandimethanol, Diethylenglykol, 5-Sulfoisophthalsäuremethylester-Na-Salz, 5-Sulfoisophthalsäure-Li-Salz, Natriumcarbonat, Titantetraisopropylat, Isophthalsäure, Polyacrylsäure und 1,4-Cyclohexandicarbonsäure vorgelegt, und langsam auf 200 °C aufgeheizt. Das dabei entstehende Methanol und Reaktionswasser werden bei Normaldruck abdestilliert. Anschließend wird ausreichend mit $N_2$ inertisiert und die Reaktionsmischung langsam auf 250 °C aufgeheizt und solange bei dieser Temperatur gehalten, bis kein Kondensat mehr überdestillliert. In einem Zeitraume von drei Stunden wird dann, von Normaldruck ausgehend, der Innendruck des Reaktionsgefäßes auf Ölpumpenvakuum abgesenkt. Nach weiteren 75 Minuten wird mit $N_2$ belüftet und die heiße Polymerschmelze ausgetragen.

Herstellungsbeispiel 6

| Edukt | Masse (g) |
|---|---|
| Pentaerythrit | 7,20 |
| 5-Sulfoisophthalsäuremethylester, Na-Salz | 15,02 |
| 5-Sulfoisophthalsäure, Li-Salz | 140,21 |
| Isophthalsäure | 55,00 |

(fortgesetzt)

| Edukt | Masse (g) |
|---|---|
| Isethionsäure, Li-Salz | 25,98 |
| Polyacrylsäure ($M_n$ = 12.000 g/mol) | 3,00 |
| Natriumcarbonat | 0,60 |
| Titantetraisopropylat | 0,60 |
| 1,4-Cyclohexandimethanol | 144,21 |
| Diethylenglycol | 400,00 |

Herstellweise:

In einem 2 l Vierhalskolben mit KPG-Rührer, Innenthermometer, Gaseinleitungsrohr und Destillationsbrücke werden Diethylenglykol, 5-Sulfoisophthalsäuremethylester-Na-Salz, 5-Sulfoisophthalsäure-Li-Salz, Natriumcarbonat, Titantetraisopropylat, Isophthalsäure, und Pentaerythrit vorgelegt, und langsam auf 200 °C aufgeheizt. Das dabei entstehende Methanol und Reaktionswasser werden bei Normaldruck abdestilliert. Danach werden die Polyacrylsäure und das Isethionsäure-Li-Salz zur Reaktionsmischung zugesetzt. Anschließend wird ausreichend mit $N_2$ inertisiert und die Reaktionsmischung langsam auf 250 °C aufgeheizt und solange bei dieser Temperatur gehalten, bis kein Kondensat mehr überdestillliert. In einem Zeitraume von drei Stunden wird dann, von Normaldruck ausgehend, der Innendruck des Reaktionsgefäßes auf Ölpumpenvakuum abgesenkt. Nach weiteren 75 Minuten wird mit $N_2$ belüftet und die heiße Polymerschmelze ausgetragen.

**(B) Rezepturbeispiele**

**<u>Beispiel 1 (W/O-Emulsion):</u>**

| | Gew.-% |
|---|---|
| PEG-7-hydriertes Ricinusöl | 4,00 |
| Wollwachsalkohol | 1,50 |
| Bienenwachs | 3,00 |
| Vaseline | 4,00 |
| Ozokerit | 4,00 |
| Paraffinöl, subliquidum | 10,00 |
| Glycerin | 15,00 |
| Octylmethoxycinnamat | 2,50 |
| Methylbenzylidencampher | 2,50 |
| Tocopherolacetat | 1,00 |
| Kammpolymer gemäß Herstellungsbeispiel 1 | 0,50 |
| Magnesiumsulfat 7 $H_2O$ | 0,70 |
| Parfüm, Konservierungsmittel, Farbstoffe, Antioxidantien etc. | q.s. |
| Wasser | ad 100,00 |

**Beispiel 2 (W/O-Emulsion):**

| | Gew.-% |
|---|---|
| PEG-7-hydriertes Ricinusöl | 4,00 |
| Wollwachsalkohol | 1,50 |
| Bienenwachs | 3,00 |
| Vaseline | 9,00 |
| Ozokerit | 4,00 |
| Paraffinöl, subliquidum | 10,00 |
| Sorbit | 15,00 |
| Kammpolymer gemäß Herstellungsbeispiel 2 | 2,50 |
| Magnesiumsulfat 7 $H_2O$ | 0,70 |
| Parfüm, Konservierungsmittel, Farbstoffe, Antioxidantien etc. | q.s. |
| Wasser | ad 100,00 |

**Beispiel 3 (W/O-Emulsion)**

| | Gew.-% |
|---|---|
| Laurylmethiconcopolyol | 1,50 |
| Cetylmethiconcopolyol | 0,50 |
| Paraffinöl, subliquidum | 10,00 |
| Cylomethicon | 2,00 |
| Dimethicon | 1,00 |
| Weizenkeimöl | 4,00 |
| Caprylsäure/Caprinsäuretriglyceride | 4,00 |
| Glycerin | 10,00 |
| Kammpolymer gemäß Herstellungsbeispiel 3 | 0,25 |
| Natriumchlorid | 1,00 |
| Parfüm, Konservierungsmittel, Farbstoffe, Antioxidantien etc. | q.s. |
| Wasser | ad 100,00 |

**Beispiel 4 (O/W-Emulsion):**

| | Gew.-% |
|---|---|
| Sorbitanmonostearat | 2,50 |
| Glycerinmonostearat | 1,00 |
| Vaseline | 0,50 |
| Paraffinöl, subliquidum | 11,00 |
| Octyldodecanol | 4,00 |
| Hydrierte Kokosfettsäureglyceride | 1,00 |
| Cyclomethicon | 1,00 |
| Carbomer | 0,15 |
| Glycerin | 10,00 |
| Tocopherolacetat | 1,00 |
| Kammpolymer gemäß Herstellungsbeispiel 4 | 0,50 |
| Parfüm, Konservierungsmittel, NaOH, Farbstoffe, Antioxidantien etc. | q.s. |
| Wasser | ad 100,00 |

**Beispiel 5 (O/W-Emulsion):**

| | Gew.-% |
|---|---|
| Stearinsäure | 1,50 |
| Sorbitanmonostearat | 0,50 |
| Myristylalkokol | 1,50 |
| Glycerinmonostearat | 0,50 |
| Paraffinöl, subliquidum | 10,00 |
| Dimethicone | 1,00 |
| Octyldodecanol | 2,00 |
| Hydrierte Kokosfettsäureglyceride | 0,50 |
| Carbomer | 0,10 |
| Serin | 0.50 |
| Glycerin | 5,00 |
| Tocopherolacetat | 0,50 |
| Kammpolymer gemäß Herstellungsbeispiel 5 | 2,50 |
| Parfüm, Konservierungsmittel, NaOH, Farbstoffe, Antioxidantien etc. | q.s. |
| Wasser | ad 100,00 |

**Beispiel 6 (Hydrodispersionsgel):**

| | Gew.-% |
|---|---|
| PEG-8 (Polythylenglycol 400) | 5,00 |
| Ethanol | 2,00 |
| Carbomer | 0,70 |
| Triglycerid, flüssig | 1,50 |
| Kammpolymer gemäß Herstellungsbeispiel 6 | 1,25 |
| Glycerin | 5,00 |
| Sorbit | 2,00 |
| Panthenol | 0,50 |
| Tocopherolacetat | 0,50 |
| Parfüm, Konservierungsmittel, NaOH, Farbstoffe, Antioxidantien etc. | q.s. |
| Wasser | ad 100,00 |

**Beispiel 7 (Hydrogel):**

| | Gew.-% |
|---|---|
| PEG-8 (Polythylenglycol 400) | 5,00 |
| Ethanol | 2,00 |
| Carbomer | 0,70 |
| Kammpolymer gemäß Herstellungsbeispiel 2 | 1,25 |
| Glycerin | 5,00 |
| Sorbit | 2,00 |
| Panthenol | 0,50 |
| Tocopherolacetat | 0.50 |
| Parfüm, Konservierungsmittel, NaOH, Farbstoffe, Antioxidantien etc. | q.s. |
| Wasser | ad 100,00 |

**Beispiel 8 (Lippenpflegegel)**

| | Gew.-% |
|---|---|
| Caprylsäure/Caprinsäuretriglyceride | 40,00 |

(fortgesetzt)

| | Gew.-% |
|---|---|
| Vaseline | 40,00 |
| Wollwachsalkohol | 1,00 |
| Bienenwachs | 3,00 |
| Kammpolymer gemäß Herstellungsbeispiel 3 | 2,50 |
| Polyisobutene | 5,00 |
| Jojoba Öl | 8,00 |
| Tocopherolacetat | 0,50 |
| Parfüm, Konservierungsmittel, Antioxidantien etc. | q.s. |

**Beispiel 9 (Gesichtspuder, gepreßt):**

| | |
|---|---|
| Magnesiumsilikat | 25,00 |
| Magnesiumstearat | 1,50 |
| Nylon-12 | 2,50 |
| Lauroyllysin | 1,00 |
| Kaolin | 10,00 |
| Magnesiumcarbonat | 5,00 |
| Kammpolymer gemäß Herstellungsbeispiel 6 | 3,50 |
| Glimmer | 5,00 |
| Eisenoxide | 1,50 |
| Titandioxid | 2,50 |
| Isopropylisostearat | 2,50 |
| Paraffinöl, subliquidum | 2,50 |
| Talkum | ad 100,00 |
| Parfüm, Konservierungsmittel, Antioxidantien etc. | q.s. |

**Beispiel 10 (Emulsions-Make-up):**

| | Gew.-% |
|---|---|
| Sorbitanmonostearat | 1,50 |
| Sorbitanmonooleat | 1,00 |
| Glycerinmonostearat | 1,00 |
| Paraffinöl, subliquidum | 7,00 |

(fortgesetzt)

| | Gew.-% |
|---|---|
| Octyldodecanol | 7,00 |
| Hydrierte Kokosfettsäureglyceride | 4,00 |
| Octylmethoxycinnamat | 2,00 |
| Butylmethoxydibenzoylmethan | 1,00 |
| Carbomer | 0,10 |
| Glycerin | 5,00 |
| 1,3 Butylenglycol | 2,00 |
| Tocopherolacetat | 1,00 |
| Kammpolymer gemäß Herstellungsbeispiel 5 | 2,50 |
| Magnesiumsilikat | 1,00 |
| Glimmer | 1,00 |
| Eisenoxide | 1,00 |
| Titandioxid | 2,50 |
| Talkum | 5,00 |
| Parfüm, Konservierungsmittel, NaOH, Farbstoffe, Antioxidantien etc. | q.s. |
| Wasser | ad 100,00 |

**Patentansprüche**

**1.** Kosmetische oder dermatologische Zubereitungen, dadurch gekennzeichnet, daß sie wasserlösliche und/oder wasserdispergierbare Kammpolymere, bestehend aus einer Polymerhauptkette und mit dieser Polymerhauptkette über Estergruppen verknüpften sulfongruppenhaltigen Polyesterseitenarmen, enthalten.

**2.** Verwendung wasserlöslicher und/oder wasserdispergierbarer Kammpolymere, bestehend aus einer Polymerhauptkette und mit dieser Polymerhauptkette über Estergruppen verknüpften sulfongruppenhaltigen Polyesterseitenarmen, zur Pflege der Haut.

**3.** Zubereitungen nach Anspruch 1 oder Verwendung nach Anspruch 2, dadurch gekennzeichnet, daß die sulfongruppenhaltigen Polyesterseitenarmen, welche wenigstens teilweise durch Natrium und Lithiumgegenionen neutralisiert wurden, wobei das molare Verhältnis von Lithium zu Natrium zwischen 0,1 und 50, bevorzugt zwischen 0,5 und 25, liegt.

**4.** Zubereitungen nach Anspruch 1 oder Verwendung nach Anspruch 2, dadurch gekennzeichnet, daß die polymere Hauptkette der Kammpolymere gewählt wird aus der Gruppe der polymeren aliphatischen, cycloaliphatischen oder aromatischen Polycarbonsäuren bzw. deren Derivaten wie beispielsweise Polyacrylsäure, Polymethacrylsäure und deren Ester (Ester der beiden Säuren mit aliphatischen, cycloaliphatischen oder aromatischen Alkoholen mit $C_1$ bis $C_{22}$), Maleinsäure, Maleinsäureanhydrid, Fumarsäure und Polynorbonensäure.

**5.** Zubereitungen nach Anspruch 1 oder Verwendung nach Anspruch 2, dadurch gekennzeichnet, daß die Kammpolymere gewählt werden aus der Gruppe der Polyester folgender genetischer Strukturformeln

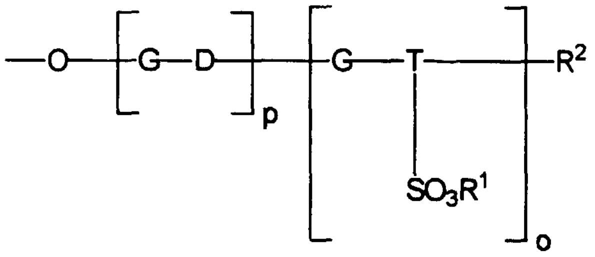

Formel I

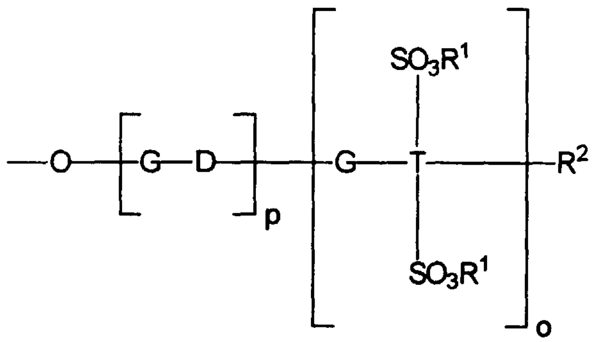

Formel II

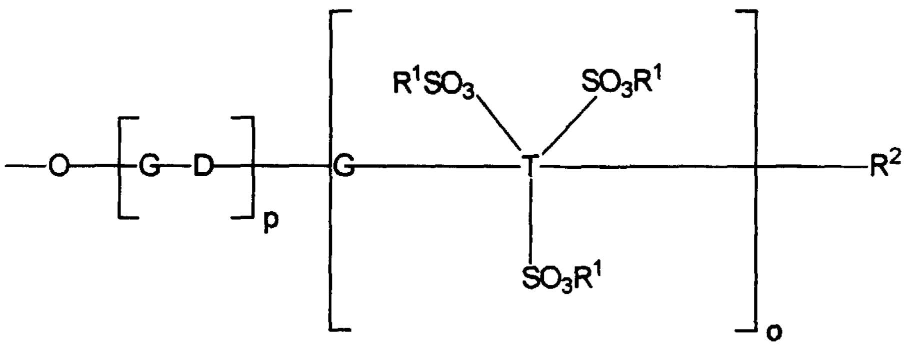

Formel III

usw.

wobei p und m so gewählt werden, daß mittlere Molekulargewichte der eingesetzten Hauptkettenbestandteile zwischen 200 und 2.000.000 g/mol liegen, wobei der Bereich von 2.000 -100.000 g/mol bevorzugt Verwendung findet, die Polyester-Seitenketten gemäß Formel I - III vorteilhaft bestehen aus:

G : einer mindestens zwei endständige Sauerstoffatome enthaltende Siloxaneinheit, die vorteilhaft durch Strukturelemente charakterisiert ist wie folgt:

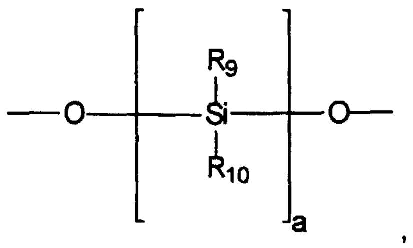

,

wobei die Siliciumatome mit gleichen oder unterschiedlichen Alkylresten und/oder Arylresten und/oder Arylakylresten substituiert werden können, welche hier verallgemeinernd durch die Reste $R_9$ - $R_{10}$ darge-

stellt sind (will sagen, daß die Anzahl der unterschiedlichen Reste nicht notwendig auf bis zu 2 beschränkt ist). a kann dabei vorteilhaft Werte von 1 - 5.000 annehmen,
sowie der mindestens zwei endständige Sauerstoffatome enthaltende aromatischen, aliphatischen oder cycloaliphatischen Organyleinheiten mit einer Kohlenstoffzahl von $C_2$ bis $C_{22}$ oder Abkömmlinge eines Polyglykols der Form HO-$[R^3\text{-}O)_k\text{-}[R^4\text{-}O)_m$-H, entsprechend einer Organyleinheit

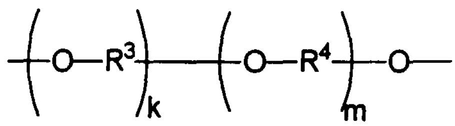

wobei die Reste $R^3$ und $R^4$ Alkylenreste darstellen mit einer Kohlenstoffzahl von $C_2$-$C_{22}$, wobei beide Reste nicht notwendigerweise verschieden sein müssen.
wobei für die Koëffzienten k und m gilt: $k+m \geq 1$, wobei k und m ferner so gewählt werden können, daß die vorab bezeichneten mittleren Molekulargewichte der eingesetzten Hauptkettenbestandteile zuwege kommen.

D : einer mindestens zwei endständige Acylgruppen enthaltenden aromatischen, aliphatischen oder cycloaliphatischen Organyleinheit mit einer Kohlenstoffzahl von $C_2$ bis $C_{22}$, wobei auch Kombinationen aus mehreren verschiedenen Säurekomponenten im beanspruchten Zielmolekül enthalten sein können, beispielsweise eine Organyleinheit des Schemas

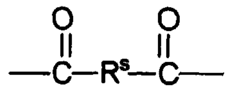

wobei $R^S$ aromatische und lineare oder cyclische, gesättigte oder ungesättigte aliphatische Bifunktionale Reste mit Kohlenstoffzahlen von $C_2$ bis $C_{22}$ darstellen kann. Auch fluorierte oder perfluorierte Acylreste sind dabei im Sinne der Erfindung
oder einer endständige Acylgruppe und eine endständige Oxogruppe enthaltenden aromatischen, aliphatischen oder cycloaliphatischen Organyleinheit mit einer Kohlenstoffzahl von $C_2$ bis $C_{22}$, wobei auch Kombinationen aus mehreren verschiedenen Säurekomponenten im beanspruchten Zielmolekül enthalten sein können, beispielsweise eine Organyleinheit des Schemas

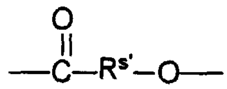

wobei $R^{S'}$ aromatische und lineare oder cyclische, gesättigte oder ungesättigte aliphatische Bifunktionale Reste mit Kohlenstoffzahlen von $C_2$ bis $C_{22}$ darstellen kann. Auch fluorierte oder perfluorierte Acylreste sind dabei im Sinne der Erfindung.

T : eine Verbindung aus der Gruppe der mindestens zwei endständige Acylgruppen enthaltenden sulfonierten aromatischen, aliphatischen oder cycloaliphatischen Organylverbindungen

$R^1$: Lithium, Natrium, Kalium, Magnesium, Calcium, Ammonium, Monoalkylammonium, Dialkylammonium, Trialkylammonium oder Tetraalkylammonium bedeuten kann, worin die Alkylpositionen der Amine unabhängig voneinander mit $C_1$ bis $C_{22}$-Alkylresten und 0 bis 3 Hydroxylgruppen besetzt sind.

$R^2$: einen Molekülrest, gewählt aus den Gruppen der

- über Etherfunktionen verbrückenden monofunktionell-linearen oder -verzweigten siliconhaltigen Organylreste,
- aromatischen, aliphatischen oder cycloaliphatischen Aminofunktionen: (-NH-$R^5$, -$NR^5_2$ wobei $R^5$

einen Alkyl- oder Arylrest mit $C_1$ bis $C_{22}$ darstellen kann)

- aromatischen, aliphatischen oder cycloaliphatischen Monocarbonsäuregruppen: ($-COOR^6$ wobei $R^6$ ein Alkyl- oder Arylrest darstellt mit $C_1$ bis $C_{200}$)
- über Etherfunktionen verbrückten aromatischen, aliphatischen oder cycloaliphatischen Organylreste: ($-O-R^5$)
- über Etherfunktionen verbrückenden Polyalkoxyverbindungen der Form

$$-O-[R^7-O]_q-[R^8-O]_r-Y$$

Die Reste $R^7$ und $R^8$ stellen vorteilhaft Alkylenreste dar mit einer Kohlenstoffzahl von $C_2$-$C_{22}$, wobei beide Reste nicht notwendigerweise verschieden sein müssen. Der Rest Y kann sowohl Wasserstoff als auch aliphatischer Natur mit $C_1$-$C_{22}$ sein. Für die Koeffizienten q und r gilt: $q+r \geq 1$.

- über Etherfunktionen verbrückenden einfach oder mehrfach ethoxylierten sulfonierten Organylreste oder bevorzugt deren Alkali- oder Erdalkalisalze, wie beispielsweise vorteilhaft gekennzeichnet durch die generische Strukturformel

$$-(O-CH_2-CH_2)_s-SO_3R^1$$

mit $s \geq 1$, und wobei s ferner so gewählt werden kann, daß die vorab bezeichneten mittleren Molekulargewichte der eingesetzen Hauptkettenbestandteile zuwege kommen.

- Siliconfunktionen, die sich von monofunktionalen Siliconen ableiten gemäß der generischen Struktformel

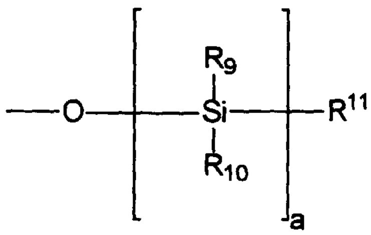

wobei $R^9$ und $R^{10}$ die genannten Eigenschaften haben, und unabhängig davon $R^{11}$ ebenfalls einen Alkylrest oder einen Arylreste oder einen Aryalkylrest darstellen kann.

**6.** Zubereitungen nach Anspruch 1 oder Verwendung nach Anspruch 2, dadurch gekennzeichnet, daß

$R^1$ : aus der Gruppe Lithium und/oder Natrium neben gegebenenfalls weiteren Gegenionen, z.B., Kalium, Magnesium, Calcium, Ammonium, Monoalkylammonium, Dialkylammonium, Trialkylammonium oder Tetraalkylammonium bedeuten kann, worin die Alkylpositionen der Amine unabhängig voneinander mit $C_1$ bis $C_{22}$-Alkylresten und 0 bis 3 Hydroxylgruppen besetzt sind,

gewählt wird.

**7.** Zubereitungen nach Anspruch 1 oder Verwendung nach Anspruch 2, dadurch gekennzeichnet, daß die mittleren Molekulargewichte der Kammpolymere vorteilhaft zwischen 200 und 2.000.000 g/mol liegen, besonders vorteilhaft zwischen 200 und 100.000 g/mol liegen wobei der Bereich von 1.000 - 30.000 g/mol bevorzugt Verwendung findet, ganz besonders vorteilhaft von 5.000 - 15.000 g/mol.

**8.** Zubereitungen nach Anspruch 1 oder Verwendung nach Anspruch 2, dadurch gekennzeichnet, daß der Gehalt an Kammpolymeren in Mengen von 0,001 - 10,0 Gew.-%, bezogen auf das Gesamtgewicht der Zubereitungen , bevorzugt 0,01 - 5,0 Gew.-%, insbesondere von 0,05 - 1,0 Gew.-%, beträgt.

**9.** Zubereitungen nach Anspruch 1 in Form von Emulsionen.